Europäisches Patentamt

European Patent Office

Office européen des brevets

(11) Numéro de publication: **0 348 309**
**A1**

## (12) DEMANDE DE BREVET EUROPEEN

(21) Numéro de dépôt: **89420201.9**

(22) Date de dépôt: **08.06.89**

(51) Int. Cl.⁴: **C 07 C 69/15**
**C 07 C 67/297**

(30) Priorité: **24.06.88 FR 8808783**

(43) Date de publication de la demande:
**27.12.89 Bulletin 89/52**

(84) Etats contractants désignés:
**AT BE DE ES FR GB GR IT NL**

(71) Demandeur: **RHONE-POULENC CHIMIE**
**25, quai Paul Doumer**
**F-92408 Courbevoie Cédex (FR)**

(72) Inventeur: **Denis, Philippe**
**16, Allée des Troenes**
**F-69150 Decines (FR)**

**Perron, Robert**
**La Pecolière**
**F-69390 Charly (FR)**

(74) Mandataire: **Varnière-Grange, Monique et al**
**RHONE-POULENC CHIMIE Service Brevets Chimie**
**Centre de Recherches des Carrières B.P. 62**
**F-69192 Saint-Fons Cédex (FR)**

(54) Procédé de fabrication de l'acétate de vinyle.

(57) La présente invention a trait à la fabrication de l'acétate de vinyle par craquage du diacétate d'éthylidène (DAE).

La présente invention a pour objet un procédé de fabrication de l'acétate de vinyle par craquage du diacétate d'éthylidène sous l'effet de la température et d'un catalyseur acide le cas échéant en présence d'anhydride acétique, caractérisé en ce que le craquage est conduit avec introduction de cétène dans le milieu réactionnel.

EP 0 348 309 A1

**Description**

## PROCEDE DE FABRICATION DE L'ACETATE DE VINYLE

La présente invention a trait à la fabrication de l'acétate de vinyle par craquage du diacétate d'éthylidène (DAE).

L'acétate de vinyle est un produit chimique dont l'importance industrielle est manifeste depuis de très nombreuses années : de grandes quantités en sont utilisées pour produire notamment des homopolymères tels que l'acétate de polyvinyle et l'alcool polyvinylique.

La synthèse de 1 acétate de vinyle (AVM) par craquage du DAE est une réaction connue depuis longtemps. De nombreux problèmes se posent dans le cadre de cette synthèse, en particulier du fait que le DAE est susceptible de se craquer ou (se décomposer) selon deux directions principales à savoir la production conjointe d'AVM et d'acide acétique ou la co-production d'acétaldéhyde et d'anhydride acétique.

Dans le brevet américain n° 2,425,389 il a été proposé de craquer le DAE dans un excès d'anhydride acétique en présence d'un acide sulfonique aromatique comme catalyseur et d'éliminer l'AVM et l'acide acétique formés par distillation au fur et à mesure de leur apparition pour favoriser le déroulement de la réaction souhaitée et limiter les riques de polymérisation et/ou de dégradation de l'AVM produit.

Il est également bien connu que le craquage du DAE en AVM est favorisé par la présence d'un grand excès d'anhydride acétique.

Toutefois l'efficacité des procédés antérieurement connus n'est pas pleinement satisfaisante, la réaction de craquage étant le plus souvent rapidement bloquée, et peu sélective comme l'atteste la proportion d'acétaldéhyde formée et, toujours accompagnée d'une coproduction d'acide acétique produit certes utile en lui-même, mais dont la production lors de la mise en oeuvre d'un tel procédé n'est pas souhaitable.

Il a maintenant été trouvé qu' il est possible de produire de l'AVM par craquage du DAE en s'affranchissant d'une co-production d'acide acétique, avec une sélectivité améliorée tout en favorisant le déroulement convenable de la réaction de craquage sur une période de temps plus compatible avec les impératifs de production à l'échelle industrielle.

La présente invention a donc pour objet un procédé de fabrication de l'acétate de vinyle par craquage du diacétate d'éthylidène sous l'effet de la température et d'un catalyseur acide, le cas échéant en présence d'anhydride acétique, caractérisé en ce que le craquage est conduit avec introduction de cétène dans le milieu réactionnel.

Le craquage est produit sous l'effet conjoint de la température et d'un catalyseur acide.

La température est typiquement comprise entre 60 et 200°C et, de préférence, entre 100 et 140°C.

La réaction en cause peut être conduite en phase liquide ou ruisselante, sous pression atmosphérique ou sous pression autogène.

Par catalyseur acide on entend n'importe quel catalyseur acide commode (supporté ou non), par exemple de l'acide sulfurique, un acide sulfonique en particulier les acides sulfoniques aromatiques tels les acides benzène -, toluène - ou naphtalène sulfonique, ou un catalyseur du type Friedel Crafts tel du fluorure de bore. L'acide sulfurique convient plus particulièrement à la mise en oeuvre du présent procédé.

La quantité de catalyseur à mettre en oeuvre n'est pas critique et le choix précis de cette quantité résultera le plus souvent pour un catalyseur donné d'un compromis entre l'activité recherchée et le coût et/ou les difficultés de manipulation de phases chargées en catalyseur acide.

Avec l'acide sulfurique de bons résultats sont obtenus pour une teneur en catalyseur comprise entre 10 et 250 mmol/l de milieu réactionnel.

Bien entendu, comme les spécialistes de ce domaine le savent bien, le procédé selon la présente invention peut être conduit en présence de quantités importantes d'anhydride acétique.

Dans le cadre du présent procédé il est essentiel que le craquage soit conduit avec introduction de cétène.

Il a en effet été constaté que la réaction de craquage ainsi conduite se déroule plus favorablement à la fois en limitant la réaction non souhaitée de craquage en acétaldéhyde et en permettant à la réaction de craquage recherchée de se dérouler sur une période plus longue, sans qu'une chute notable de productivité soit observée.

Par ailleurs l'introduction de cétène permet de s'affranchir totalement de la co-production d'acide acétique et de faciliter la séparation et la récupération de l'acétate de vinyle.

L'introduction de cétène peut être réalisée de diverses manières. Ainsi on peut introduire le cétène au fur et à mesure que la réaction de craquage se déroule ou bien en continu, ce qui en pratique se révèle plus commode.

Le débit de cétène peut bien entendu varier dans de larges limites. De bons résultats sont obtenus lorsque ce débit, exprimé en mole par heure et par mole de DAE à convertir, est compris entre 0,5 et 10 et, de préférence entre 1 et 5.

Le procédé selon la présente invention peut être mis en oeuvre en discontinu ou en continu. Après la durée souhaitée pour la raction (ou le temps de séjour fixé) on sépare les constituants du mélange réactionnel par tout moyen approprié, par distillation par exemple et on récupère l'acétate de vinyle.

Les exemples ci-après illustrent l'invention : Mode opératoire :

Dans un autoclave en verre muni d'une agitation magnétique et d'une arrivée de gaz, surmonté par une colonne Vigreux dont la tête est reliée à des pièges montés en série, on introduit une solution de diacétate d'éthylidène et d'acide sulfurique dans l'anhydride acétique. L'agitation est mise en route ; la solution est amenée et maintenue au reflux (140°C). Le cétène est alors mis à buller dans la solution à débit constant. Après la durée réactionnelle souhaitée, le chauffage l'agitation et l'introduction de gaz sont arrêtés. Les produits sont alors analysés par chromatographie en phase gazeuse, étant précisé que l'acétaldéhyde et de l'anhydride acétique se retrouvent dans les pièges disposés en aval du réacteur et que le diacétate d'éthylidène, de l'anhydride acétique et, le cas échéant de l'acide acétique se trouvent dans le réacteur de craquage.

EXEMPLES 1 A 3 ; Essai témoin a :

Selon le mode opératoire décrit ci-avant on réalise une série d'essais en utilisant des débits différents de cétène sur une charge renfermant :
- 50 mmol de diacétate d'éthylidène
- 53 mmol d'anhydride acétique et
- 100 mmol/l d'acide sulfurique.

La durée de chaque essai est de 30 minutes. Les conditions particulières ainsi que les résultats obtenus figurent dans le tableau I ci-après :

TABLEAU I

| Réf. | Cétène mmol/h | DAE mmol | AC2O mmol | AVM mmol | AcH mmol |
|---|---|---|---|---|---|
| ( a ) | 0 | 38 | 58 | 2,6 | 2,1 |
| 1 | 50 | 33 | 68 | 5,8 | 3,4 |
| 2 | 95 | 34 | 74 | 5,5 | 4,0 |
| 3 | 250 | 14 | 81 | 8,6 | 6,6 |

Dans l'essai témoin (a) il y a production d'acide acétique. Dans les exemples 1 à 3 on n'en détecte pas.

Essai témoin b :

On reproduit l'essai témoin (a) ci-avant en analysant les produits formés respectivement au bout de 20, 40 et 75 minutes de réaction. Les résultats sont rapportés au tableau II ci-après (L'acide acétique formé n'est pas dosé).

TABLEAU II

| Durée de réaction en minutes | DAE mmol | AC2O mmol | AVM mmol | ACH mmol |
|---|---|---|---|---|
| 20 | 39 | 60 | 3 | 1,7 |
| 40 | 36 | 57 | 2,5 | 2 |
| 75 | 34 | 61 | 3,5 | 3,5 |

Ces résultats montrent que la réaction de production d'AVM est pratiquement bloquée au bout de 20 minutes environ.

EXEMPLE 4 :

On reproduit l'exemple 2 ci-avant en analysant les produits formés respectivement au bout de 20, 40 et 75 minutes de réaction.

Les résultats sont rapportés au tableau III ci-après, étant précisé qu'on ne détecte pas d'acide acétique.

TABLEAU III

| Durée de réaction en minutes | DAE mmol | AC2O mmol | AVM mmol | ACH mmol |
|---|---|---|---|---|
| 20 | 40 | 70 | 4,4 | 2,2 |
| 40 | 28 | 79 | 6,9 | 6,3 |
| 75 | 17 | 87 | 13,3 | 10,7 |

EXEMPLE 5 :

On reproduit l'exemple 2 ci-avant en multipliant la quantité de catalyseur par deux et en adoptant comme durée de réaction 20 minutes. On détecte la formation de 6,8 mmol d'AVM et de 3,2 mmol d'AcH.

**Revendications**

1° - Procédé de fabrication de l'acétate de vinyle par craquage du diacétate d'éthylidène sous l'effet de la température et d'un catalyseur acide le cas échéant en présence d'anhydride acétique, caractérisé en ce que le craquage est conduit avec introduction de cétène dans le milieu réactionnel.

2° - Procédé selon la revendication 1, caractérisé en ce que le débit de cétène introduit est compris entre 0,5 et 10 mole par heure et par mole de DAE à tranformer.

3° - Procédé selon la revendication 2 caractérisé en ce que ledit débit est compris entre 1 et 5 mole par heure et par mole de DAE à transformer.

4° - Procédé selon l'une quelconque des revendications précédentes caractérisé en ce que le catalyseur est l'acide sulfurique.

5° - Procédé selon l'une quelconque des revendications précédentes caractérisé en ce que la température de craquage est comprise entre 100 et 140°C.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | US-A-2 860 159 (S.P. SHARP) <br> * Colonnes 5-6; revendications * <br> --- | 1 | C 07 C 69/15 <br> C 07 C 67/297 |
| A | EP-A-0 068 795 (MITSUBISHI GAS CHEMICAL CO.) <br> * Pages 13-14; revendications * <br> --- | 1 | |
| A | FR-A-2 413 354 (HALCON RESEARCH AND DEVELOPMENT CORP.) <br> * Page 15, ligne 31 - page 16, ligne 8; page 30, revendications * <br> ----- | 1 | |

| | DOMAINES TECHNIQUES RECHERCHES (Int. Cl.4) |
|---|---|
| | C 07 C 69/00 <br> C 07 C 67/00 |

Le présent rapport a été établi pour toutes les revendications

| Lieu de la recherche | Date d'achèvement de la recherche | Examinateur |
|---|---|---|
| LA HAYE | 28-08-1989 | KINZINGER J.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

............................................................

& : membre de la même famille, document correspondant

EPO FORM 1503 03.82 (P0402)